# EUROPEAN PATENT APPLICATION

(11) **EP 3 607 906 A1**
(43) Date of publication of application: **12.02.2020**
(21) Application number: 19191078.5
(22) Date of filing: 09.08.2019
(51) Int. Cl.: A61B 34/20

(54) **IDENTIFICATION AND NOTIFICATION OF TOOL DISPLACEMENT DURING MEDICAL PROCEDURE**

(30) Priority: 10.08.2018 US 201816100277
(71) Applicant: Covidien LP, Mansfield, MA 02048 (US)
(72) Inventor: KRIMSKY, William S., Forest Hill, MD Maryland 21050 (US); STOPEK, Joshua B., Minneapolis, Minnesota 55419 (US)
(74) Representative: Soames, Candida Jane

(57) **Abstract**

The present disclosure relates to systems, devices and methods for identifying displacement of a surgical tool during a surgical procedure. An illustrative surgical system includes a surgical tool, an electromagnetic (EM) sensor, an EM navigation system including an EM field generator configured to generate an EM field and an EM tracking system configured to track the EM sensor within the EM field, and a computing device including a processor and a memory storing instructions which, when executed by the processor, cause the computing device to receive a position of a target within the EM field, receive tracking data from the EM tracking system regarding a position of the EM sensor, determine that the surgical tool is placed proximate the target, determine that the EM sensor has moved after the surgical tool is placed proximate the target, and provide an alert.

## Description

### BACKGROUND

### Technical Field

The present disclosure relates to tools for assisting surgeons during performance of medical procedures, and more particularly, to systems and methods for identifying and providing notifications of tool displacement during medical procedures.

### Description of Related Art

Surgical tools, such as catheters, needles, antennae, biopsy tools, etc., are commonly used during various surgical procedures. Various such tools include sensors to assist with determining the position of the tools within a patient's body and to assist surgeons in placing the tools at a desired location within the patient's body. However, the patient's body is never perfectly still, even when the patient is sedated, and even relatively minor movements, such as due to breathing, heartbeat, etc., may cause displacement of the tools and/or cause the pose of the patient's body relative to the tools to change even if the surgeon is not actively moving the tools. Disclosed hereinbelow are systems and methods for detecting such passive movement and/or displacement of the tools and providing notifications to the surgeon regarding the detection and recommended procedures to verify the position of the tools.

### SUMMARY

Provided in accordance with embodiments of the present disclosure are systems for identifying displacement of surgical tools during a surgical procedure. In an aspect of the present disclosure, an illustrative system includes a surgical tool, an electromagnetic (EM) sensor, an EM navigation system including an EM field generator configured to generate an EM field and an EM tracking system configured to track the EM sensor within the EM field, and a computing device including a processor and a memory storing instructions which, when executed by the processor, cause the computing device to receive a position of a target within the EM field, receive tracking data from the EM tracking system regarding a position of the EM sensor, determine that the surgical tool is placed proximate the target, determine that the surgical tool has moved after the surgical tool is placed proximate the target, and provide an alert.

In another aspect, the instructions, when executed by the processor, further cause the computing device to determine that the movement of the surgical tool is greater than a predetermined threshold, and cause a display device to display guidance for confirming placement of the surgical tool.

In a further aspect, the guidance for confirming placement of the surgical tool includes instructions to acquire image data of at least a portion of the patient's body.

In another aspect, the guidance for confirming placement of the surgical tool includes instructions to reposition the surgical tool.

In yet another aspect, the surgical tool is an extended working channel (EWC), and wherein the guidance for confirming placement of the surgical tool includes instructions to remove a treatment tool from the EWC and insert a tool including a second EM sensor into the EWC.

In still another aspect, the predetermined threshold is based on a type of surgical procedure being performed.

In yet another aspect, the predetermined threshold is based on critical structures proximate the target.

In still another aspect, the EM sensor is coupled to the surgical tool, and determining that the surgical tool is placed proximate the target includes determining that the EM sensor is proximate the position of the target.

In yet another aspect, the EM sensor is coupled to the surgical tool, and determining that the surgical tool has moved after the surgical tool is placed proximate the target includes determining that the EM sensor has moved after the surgical tool is placed proximate the target.

In still another aspect, the EM sensor is positioned on the patient's chest.

In yet another aspect, the system further includes an extended working channel (EWC), the EM sensor is coupled to the EWC, the surgical tool is inserted into the EWC, and determining that the surgical tool is placed proximate the target includes determining that the EM sensor is proximate the position of the target.

In still another aspect, determining that the surgical tool has moved after the surgical tool is placed proximate the target includes determining that the EM sensor has moved after the surgical tool is placed proximate the target.

In yet another aspect, the instructions, when executed by the processor, further cause the computing device to receive image data of at least a portion of a patient's body, cause a display device to display the image data, and cause the display device to display an indication of the position of the EM sensor on the image data.

In still another aspect, the instructions, when executed by the processor, further cause the computing device to receive image data of at least a portion of a patient's body, generate a three-dimensional (3D) model of the portion of the patient's body based on the image data, cause a display device to display the 3D model, and cause the display device to display an indication of the position of the EM sensor on the 3D model.

In a further aspect, the instructions, when executed by the processor, further cause the computing device to cause a display device to display an indication of movement of the surgical tool when it is determined that the surgical tool has moved after the surgical tool is placed proximate the target.

In another aspect, the instructions, when executed by the processor, further cause the computing device to cause the display device to display an indication of the position of the target on the 3D model, and cause the display device to display an indication of a distance between a position of the surgical tool and the position of the target.

In a further aspect, the position of the surgical tool is determined based on the position of the EM sensor.

In another aspect, the position of the surgical tool is determined based on image data.

In yet another aspect, providing an alert includes causing a display device to display a visual alert.

In still another aspect, providing an alert includes emitting an audible alert.

In yet another aspect, determining that the surgical tool has moved after the surgical tool is placed proximate the target includes determining that the movement of the surgical tool is greater than a predetermined threshold.

Provided in accordance with embodiments of the present disclosure are methods for identifying displacement of surgical tools during a surgical procedure. In an aspect of the present disclosure, an illustrative method includes receiving a position of a target within an electromagnetic (EM) field, receiving tracking data from an EM tracking system regarding a position of an EM sensor, determining that a surgical tool is placed proximate the target, determining that the EM sensor has moved after the surgical tool is placed proximate the target, and providing an alert.

Provided in accordance with embodiments of the present disclosure are non-transitory computer-readable storage media storing instructions which, when executed by a processor, cause a computing device to receive a position of a target within an electromagnetic (EM) field, receive tracking data from an EM tracking system regarding a position of an EM sensor, determine that a surgical tool is placed proximate the target, determine that the EM sensor has moved after the surgical tool is placed proximate the target, and provide an alert.

Any of the above aspects and embodiments of the present disclosure may be combined without departing from the scope of the present disclosure.

### BRIEF DESCRIPTION OF THE DRAWINGS

Various aspects and features of the present disclosure are described hereinbelow with references to the drawings, wherein:
FIG. 1 is a schematic diagram of an endobronchial system for planning and performing treatment of an area of a patient's lungs, according to an embodiment of the present disclosure;
FIG. 2 is a block diagram of an example computing device forming part of the system of FIG. 1, according to an embodiment of the present disclosure;
FIGs. 3A and 3B show a flowchart of an example method for providing visual guidance for navigating inside a patient's chest, according to an embodiment of the present disclosure;
FIG. 4 shows an exemplary user interface which may be presented as part of the method of FIGs. 3A and 3B;
FIG. 5 shows an example of how a tool forming part of the system of FIG. 1 may be displaced, according to an embodiment of the present disclosure;
FIGs. 6A and 6B show other exemplary user interfaces which may be presented as part of the method of FIGs. 3A and 3B; and
FIGs. 7A and 7B show other examples of how a tool forming part of the system of FIG. 1 may be displaced, according to an embodiment of the present disclosure.

### DETAILED DESCRIPTION

The present disclosure generally relates to devices, systems, and methods for identifying tool displacement during a medical procedure, and for providing notifications and instructions to verify tool placement when displacement is detected. More particularly, the present disclosure relates to detecting displacement of surgical tools placed at a treatment site within a patient's body due to movement of the tool and/or movement of the patient's body relative to the tool, and providing notifications and instructions to a surgeon regarding the displacement and potential procedures to verify the position of the tool.

Surgical navigation tools, such as catheters, are used during surgical procedures to assist in placing other tools, such as biopsy tools, ablation tools, etc., at a treatment site within a patient's body. A variety of catheters have been developed for use during medical procedures. Most catheters are at least modestly flexible to allow the catheters to be navigated through a tortuous luminous network, such as the bronchial, vascular, or other luminal networks within a patient's body. Many catheters include one or more position sensors at a distal portion and/or along a length of the catheters. Additionally or alternatively, the position sensors may be included in a tool inserted through the catheters.

The position of the catheter within three-dimensional space, and a corresponding position within the patient's body, may be determined based on the position sensors. Thus, a surgeon may navigate a catheter to a treatment site in a patient's body by viewing the determined position of the catheter via a navigation system, such as the system described below. However, even after a catheter is placed at a treatment site, the position of the catheter, and particularly the distal portion of the catheter, may continue to move, such as by insertion of tools into the catheter, or even without active movement caused by the surgeon. Further, the position of the patient's body relative to the position of the catheter may move, due to natural movement cause by the patient's respiratory and/or cardiac cycles, or other causes such as coughing or physical movement of the patient.

In conventional systems, the surgeon is left to estimate whether movement of the catheter and/or the patient after the catheter is placed would have moved the distal portion of the catheter away from the treatment site. However, as described further below, the present disclosure provides for systems and methods for detecting displacement of a distal portion of a catheter after placement at a treatment site, determining whether the displacement is significant enough to require repositioning of the catheter, and providing notifications and/or instructions to the surgeon regarding the detected displacement and procedures to confirm the placement of the catheter. To that end, and as described in further detail below, various models of the patient's body may be generated to assist the surgeon during placement of the catheter at the treatment site, detect displacement of the catheter after it is placed at the treatment site, determine whether the detected displacement of the catheter has moved the catheter away from the treatment site, and to further allow the clinician to visualize the position of the catheter following displacement.

In some embodiments, image data may be acquired to generate, and be displayed in conjunction with or alongside, a digital reconstruction, such as a three-dimensional (3D) model or map, of the patient's lungs or another portion of the patient's body. Various imaging modalities may be used to acquire the image data, including computed tomography (CT) imaging, cone beam computed tomography (CBCT) imaging, magnetic resonance (MR) imaging, positron emission tomography (PET) imaging, fluoroscopic imaging, and X-ray imaging, and/or ultrasound imaging. The 3D model may be constructed based on preoperative image data from one or more of the aforementioned imaging modalities. Alternatively or in addition, additional image data, such as from a CBCT scan, may be acquired at the start of the treatment procedure, which may also be used for registration purposes as further described below, may further be used for constructing and/or enhancing the 3D model.

To create the 3D model, a preoperative segmental and subsegmental delineation and extrapolation may be performed based on image data of the patient's lungs to create a visual representation of the patient's lungs, including lumens, pleural surfaces and fissures of the patient's lungs, and/or tumors or other aberrant structures that may be present in the patient's lungs. The delineation may be performed using one or more software applications executing on a computer. The application may generate the 3D model of the patient's lungs based on the image data, noted above, to use for the visual representation of the patient's lungs. The 3D model and image data may then be viewed by a clinician and/or surgeon to plan a medical treatment procedure, such as a surgical or interventional procedure. The 3D model and/or treatment plan may further be stored for later viewing during the treatment procedure in an operating room or the like.

As described further below, the treatment plan may include identified locations for one or more treatment targets, such as tumors, lesions, or other aberrant structures identified in the image data, and a pathway between the patient's trachea and each of the treatment targets. The pathway may include a portion located inside lumens, such as airways, of the patient's lungs, and a portion located outside of the airways of the patient's lungs. An "exit point" may mark the transition point between the portion of the pathway located inside the patient's airways and the portion of the pathway located outside of the patient's airways.

During the treatment procedure, the 3D model may be displayed, as further described below, to assist the clinician in navigating one or more tools to the treatment target. The 3D model may include an indicator of a tracked position of the tool inside the patient's lungs. At various times during the treatment procedure, additional image data may be acquired, such as by performing additional CBCT scans, to show a real-time location of the tool and/or the treatment target in the patient's lungs. For example, after the tool passes the "exit point" and is located outside of the patient's airways, or at any other time of the clinician's choosing, additional image data may be acquired and processed to identify the tool and/or the treatment target. The indicator on 3D model of the tracked position of the tool may then be updated based on the additional image data, thereby showing a confirmed location of the tool and/or the treatment target. The additional image data may further show, and thus provide a software application the ability to track, the location of the tool during various phases of the patient's respiration cycle. While the 3D model may be generated based on image data acquired while the patient was in a particular phase of the respiration cycle, e.g. full breath hold, the patient will not remain in that phase of the respiration cycle for the entire duration of the treatment procedure. Thus, acquiring image data during the treatment procedure, during various phases of the patient's respiration cycle, particularly during normal tidal volume breathing, may provide a clearer and more accurate visualization of the location of the tool and the treatment target inside the patient's lungs, as well as the position of the tool relative to the treatment target.

Further, as will be appreciated by those skilled in the art, the devices, systems, and methods described herein may also be used during other types of medical procedures, such as percutaneous and/or laparoscopic procedures, involving placement of a tool at a treatment site under image-guided and/or electromagnetic (EM) systems. As such, the illustrative embodiments described below are merely provided as examples, and are not intended to be limiting.

An electromagnetic navigation (EMN) system, such as the ELECTROMAGNETIC NAVIGATION BRONCHOSCOPY system currently sold by Medtronic PLC under the brand name SUPERDIMENSION, may be used for planning and performing treatment of an area of a patient's lungs. Generally, in an embodiment, the EMN system may be used in planning treatment of an area of the patient's lungs by identifying the positions of one or more treatment targets in the patient's lungs, selecting one or more of the treatment targets as a target location, determining a pathway to the target location, navigating a positioning assembly to the target location, and navigating a variety of tools to the target location via the positioning assembly. The EMN system may be configured to display various views of the patient's lungs, including the aforementioned image data and 3D model.

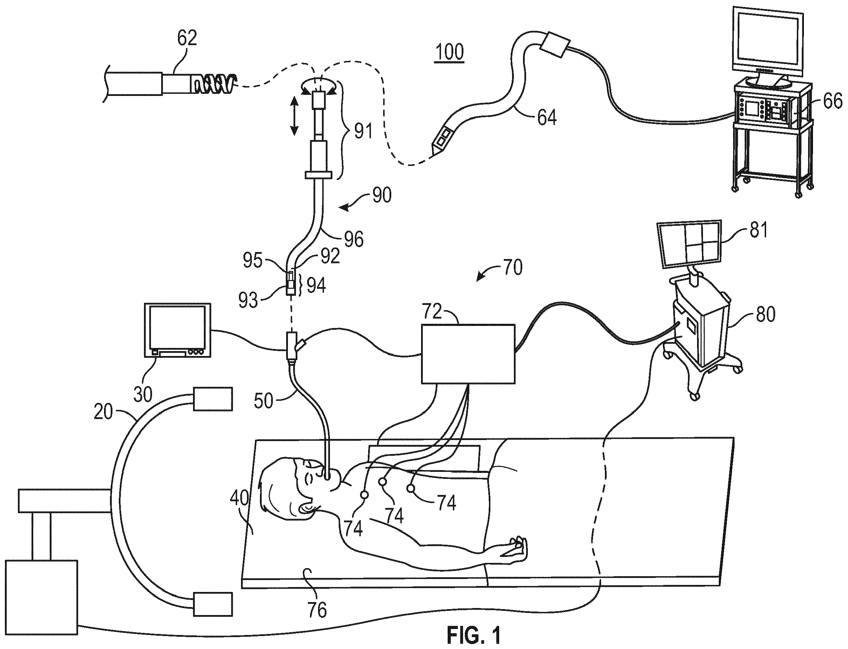

With reference to FIG. 1, an electromagnetic navigation (EMN) system 100 suitable for implementing methods for detecting tool displacement during medical procedures is provided in accordance with the present disclosure. As shown in FIG. 1, EMN system 100 is used to perform one or more procedures on a patient supported on an operating table 40. In this regard, EMN system 100 generally includes a bronchoscope 50, monitoring equipment 30, an electromagnetic (EM) tracking system 70, and a computing device 80.

Bronchoscope 50 is configured for insertion through the patient's mouth and/or nose into the patient's airways. Bronchoscope 50 includes a source of illumination and a video imaging system (not explicitly shown) and is coupled to monitoring equipment 30, for example, a video display, for displaying the video images received from the video imaging system of bronchoscope 50. In an embodiment, bronchoscope 50 may operate in conjunction with a catheter guide assembly 90. Catheter guide assembly 90 includes an extended working channel (EWC) 96 configured for insertion through a working channel of bronchoscope 50 into the patient's airways (although the catheter guide assembly 90 may alternatively be used without bronchoscope 50). Catheter guide assembly 90 further includes a handle 91 connected to EWC 96, and which can be manipulated by rotation and compression to steer EWC 96 and/or tools inserted through EWC 96, such as a locatable guide (LG) 92. EWC 96 is sized for placement into the working channel of bronchoscope 50. In the operation of catheter guide assembly 90, LG 92, including an EM sensor 94, is inserted into EWC 96 and locked into position such that EM sensor 94 extends a desired distance beyond a distal tip 93 of EWC 96. In some embodiments, EWC 96 may also include or have coupled thereto an EM sensor 95, and the EM sensors 94, 95 may alternately or collectively be used to determine the position of and steer EWC 96 and/or tools inserted therein. The location of EM sensors 94, 95, and thus distal tip 93 of EWC 96, within an EM field generated by EM field generator 76, can be derived by tracking module 72 and computing device 80. For a more detailed description of catheter guide assembly 90, reference is made to commonly-owned U.S. Patent No. 9,247,992, entitled "MICROWAVE ABLATION CATHETER AND METHOD OF UTILIZING THE SAME", filed on March 15, 2013, by Ladtkow et al., the entire contents of which are hereby incorporated by reference.

LG 92 and EWC 96 are selectively lockable relative to one another via a locking mechanism. A six degrees-of-freedom EM tracking system 70, e.g., similar to those disclosed in U.S. Patent No. 6,188,355, entitled "WIRELESS SIX-DEGREE-OF-FREEDOM LOCATOR", filed on December 12,1997, by Pinhas Gilboa, U.S. Patent No. 6,833,814, entitled "INTRABODY NAVIGATION SYSTEM FOR MEDICAL APPLICATIONS", filed on August 2, 1998, by Gilboa et al., and PCT Publication No. WO/2001/067035, entitled "OBJECT TRACKING USING A SINGLE SENSOR OR A PAIR OF SENSORS", filed on March 9, 2000, by Pinhas Gilboa, the entire contents of each of which are incorporated herein by reference, or any other suitable positioning measuring system, is utilized for performing navigation, although other configurations are also contemplated.

EM tracking system 70 may be configured for use with catheter guide assembly 90 to track a position of EM sensors 94, 95 as they moves in conjunction with EWC 96 through the airways of the patient, as detailed below. In an embodiment, EM tracking system 70 includes a tracking module 72, a plurality of reference sensors 74, and an EM field generator 76. As shown in FIG. 1, EM field generator 76 is positioned beneath the patient. EM field generator 76 and the plurality of reference sensors 74 are interconnected with tracking module 72, which derives the location of each reference sensor 74 in the six degrees of freedom. One or more of reference sensors 74 are attached to the chest of the patient. The six degrees of freedom coordinates of reference sensors 74 are sent as data to computing device 80, which includes an application 81, where the data from reference sensors 74 are used to calculate a patient coordinate frame of reference.

Although EM sensors 94, 95 are described above as being included in LG 92 and EWV 96, respectively, it is also envisioned that an EM sensor may be embedded or incorporated within a treatment tool, such as a biopsy tool 62 and/or an ablation tool 64, where the treatment tool may alternatively be utilized for navigation without need of LG 92 or the necessary tool exchanges that use of LG 92 requires. In addition to biopsy tools 62 and ablation tools 64, those skilled in the art will appreciate that various other tools, such as cameras and other optical devices, as well as other types of sensing tools, may also be used without departing from the scope of the present disclosure.

According to an embodiment, treatment tools 62, 64 are configured to be insertable into catheter guide assembly 90 and EWC 96 following navigation to a target location and removal of LG 92 (if used). Biopsy tool 62 may be used to collect one or more tissue samples from the target location, and in an embodiment, is further configured for use in conjunction with tracking system 70 to facilitate navigation of biopsy tool 62 to the target location, and tracking of a location of biopsy tool 62 as it is manipulated relative to the target location to obtain the tissue sample. Ablation tool 64 is configured to be operated with a generator 66, such as a radio frequency generator or a microwave generator, and may include any of a variety of ablation tools and/or catheters, examples of which are more fully described in U.S. Patent No. 9,259,269, entitled "MICROWAVE ABLATION CATHETER AND METHOD OF USING THE SAME", filed on March 15, 2013, by Ladtkow et al., the entire contents of which are incorporated herein by reference. Though shown as a biopsy tool and microwave ablation tool in FIG. 1, those of skill in the art will recognize that other tools including for example RF ablation tools, brachytherapy tools, cryo-ablation tools, and others may be similarly deployed and tracked without departing from the scope of the present disclosure. Additionally, a piercing tool and/or puncture tool may be used and/or incorporated within LG 92 to create an exit point where LG 92, and thereby EWC 96, is navigated outside of the patient's airways and toward the target location, as further described below.

In some embodiments, a radiographic imaging device 20, such as a computed tomography (CT) imaging device, magnetic resonance imaging (MRI) imaging device, positron emission tomography (PET) imaging device, a cone beam computed tomography (CBCT) imaging device such as a C-arm imaging device, and/or any other imaging device capable of performing a scan of at least a portion of the patient's lungs, may be used in conjunction with EMN system 100. Imaging device 20 may further be capable of performing fluoroscopic scans of the patient's lungs. As shown in FIG. 1, imaging device 20 is connected to computing device 80 such that application 81 may receive and process image data obtained by imaging device 20. However, imaging device 20 may also have a separate computing device located within the treatment room or in a separate control room to first receive the image data obtained by imaging device 20 and relay such image data to computing device 80. For example, to avoid exposing the clinician to unnecessary radiation from repeated radiographic scans, the clinician may exit the treatment room and wait in an adjacent room, such as the control room, while imaging device 20 performs the scan.

Computing device 80 includes software and/or hardware, such as application 81, used to facilitate the various phases of an EMN procedure, including generating a 3D model, identifying a target location, planning a pathway to the target location, registering the 3D model with the patient's actual airways, navigating to the target location, and performing treatment at the target location. For example, computing device 80 utilizes data acquired from a CT scan, CBCT scan, MRI scan, PET scan, and/or any other suitable imaging modality to generate and display the 3D model of the patient's airways, to enable identification of a target location on the 3D model (automatically, semi-automatically or manually) by analyzing the image data and/or 3D model, and allow for the determination and selection of a pathway through the patient's airways to the target location. The 3D model may be presented on a display monitor associated with computing device 80, or in any other suitable fashion. An example of the planning software described herein can be found in U.S. Patent No. 9,459,770, entitled "PATHWAY PLANNING SYSTEM AND METHOD", filed on March 15, 2013, by Baker et al., the contents of which are incorporated herein by reference. Further examples of the planning software can be found in commonly assigned U.S. Patent No. 9,770,216, entitled "SYSTEM AND METHOD FOR NAVIGATING WITHIN THE LUNG", filed on June 29, 2015, by Brown et al., the contents of which are incorporated herein by reference.

Using computing device 80, various views of the image data and/or 3D model may be displayed to and manipulated by a clinician to facilitate identification of the target location. As noted above, the target location may be a site within the patient's lungs where treatment is to be performed. For example, the treatment target may be located in lung tissue adjacent to an airway. The 3D model may include, among other things, a model airway tree corresponding to the actual airways of the patient's lungs, and show the various passages, branches, and bifurcations of the patient's actual airway tree. Additionally, the 3D model may include representations of lesions, markers, blood vessels and vascular structures, lymphatic vessels and structures, organs, other physiological structures, and/or a 3D rendering of the pleural surfaces and fissures of the patient's lungs. Some or all of the aforementioned elements may be selectively displayed, such that the clinician may choose which elements should be displayed when viewing the 3D model.

After identifying the target location, application 81 may determine a pathway between the patient's trachea and the target location via the patient's airways. In instances where the target location is located in lung tissue that is not directly adjacent an airway, at least a portion of the pathway will be located outside of the patient's airways to connect an exit point on an airway wall to the target location. In such instances, LG 94 and/or EWC 96 will first be navigated along a first portion of the pathway through the patient's airways to the exit point on the airway wall. LG 94 may then be removed from EWC 96 and an access tool, such as a piercing or puncture tool, inserted into EWC 96 to create an opening in the airway wall at the exit point. EWC 96 may then be advanced through the airway wall into the parenchyma surrounding the airways. The access tool may then be removed from EWC 96 and LG 94 and/or tools 62, 64 reinserted into EWC 96 to navigate EWC 96 along a second portion of the pathway outside of the airways to the target location.

During a procedure, EM sensors 94, 95, in conjunction with tracking system 70, enables tracking of EM sensors 94, 95 (and thus distal tip 93 of EWC 96 or tools 62, 64) as EWC 96 is advanced through the patient's airways following the pathway planned during the planning phase. As an initial step of the procedure, the 3D model is registered with the patient's actual airways to enable application 81 to display an indication of the position of EM sensors 94, 95 on the 3D model corresponding to the location of EM sensors 94, 95 within the patient's airways.

One potential method of registration involves performing a survey of the patient's lungs by navigating LG 92 into each lobe of the patient's lungs to at least the second bifurcation of the airways of that lobe. The position of LG 92 is tracked during this registration phase, and the 3D model is iteratively updated based on the tracked position of LG 92 within the actual airways of the patient's lungs. Such a registration process is further described in commonly-owned U.S. Patent Application Publication No. 2011/0085720, entitled "AUTOMATIC REGISTRATION TECHNIQUE", filed on May 14, 2010, by Dorian Averbuch, and U.S. Patent Application Publication No. 2016/0000356, entitled "REAL-TIME AUTOMATIC REGISTRATION FEEDBACK", filed on July 2, 2015, by Brown et al., the contents of each of which are incorporated herein by reference. While the registration process focuses on aligning the patient's actual airways with the airways of the 3D model, registration also ensures that the position of vascular structures, pleural surfaces, and fissures of the lungs are accurately determined.

Another potential method of registration uses image data from a CBCT scan performed at the start of the treatment procedure to generate the 3D model with the patient remaining on table 40 while the clinician performs the above-described planning phase. Because the scan is taken with reference sensors 74 placed on the patient, the anatomy of the patient relative to reference sensors 74 is known. By performing the scan with reference sensors 74 placed on the patient, performing registration by using the lung survey technique, described above, becomes unnecessary. Additionally, features and sensors in EM field generator 76 under the patient may also be used as another means to help ensure the target location is placed within the EM field. The clinician may then start the navigation phase of the procedure without performing the above-described survey of the patient's lungs because the patient will still be in substantially the same position as the patient was when the image data on which the 3D model is based were obtained. Thus, application 81 may extrapolate sufficient data points from the position of EM sensors 94, 95 within the EM field while LG 92 and/or EWC 96 is navigated along the planned pathway to register the 3D model to the patient's actual airways while the navigation phase is in process.

At various times during the procedure, the clinician may request that additional image data to be acquired, and thus additional scans, for example fluoroscopic and/or CBCT scans, be performed on the patient to confirm the location of LG 92, EWC 96, and/or tool 62, 64. The additional scans may be directed at a particular location in the patient's body, such as an area of the patient's lungs about the position of LG 92, EWC 96, and/or tool 62, 64, for which the clinician wants additional image data. The additional image data may thus be used to confirm the position of EM sensors 94, 95 (representing the location of LG 92, EWC 96, and/or tool 62, 64) and/or the target location within the patient's lungs. Application 81 may receive the additional image data acquired by the additional scan and process the additional image data to identify the position of LG 92, EWC 96, tool 62, 64 and/or the target location within the patient's lungs. Application 81 may then update the indicator of the position of EM sensors 94, 95 on the 3D model based on the additional image data if the additional image data indicates that the position displayed based on the original image data is incorrect. In some embodiments, the additional scans may be performed based on the patient's breathing or respiratory cycle, such as to acquire image data during different phases of the patient's respiratory cycle, as further described below. Image data acquired from fluoroscopic scans may further be used to assist the clinician in navigating and positioning LG 92, EWC 96, and/or tool 62, 64 about the target location.

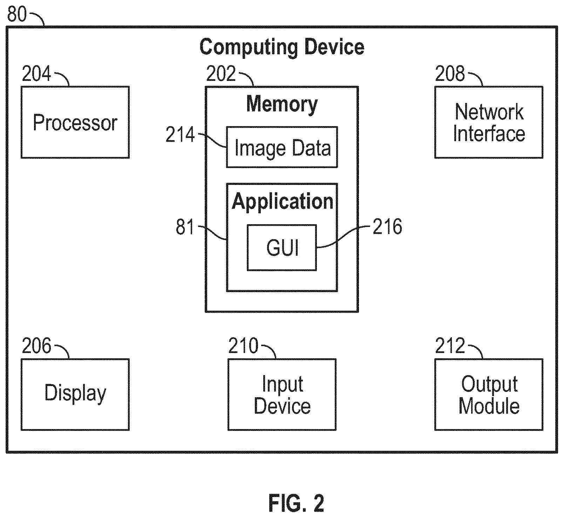

Turning now to FIG. 2, there is shown a simplified block diagram of computing device 80. Computing device 80 may include a memory 202, a processor 204, a display 206, a network interface 208, an input device 210, and/or an output module 212. Memory 202 may store application 81 and/or image data 214. Application 81 may include instructions and/or executable code for generating a graphical user interface (GUI) 216 which, when executed by processor 204, cause display 206 to display a GUI.

Memory 202 may include any non-transitory computer-readable storage media for storing data and/or software that is executable by processor 204 and which controls the operation of computing device 80. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 80.

Network interface 208 may be configured to connect to a network such as a local area network (LAN) consisting of a wired network and/or a wireless network, a wide area network (WAN), a wireless mobile network, a Bluetooth network, and/or the internet. Input device 210 may be any device by means of which a user may interact with computing device 80, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

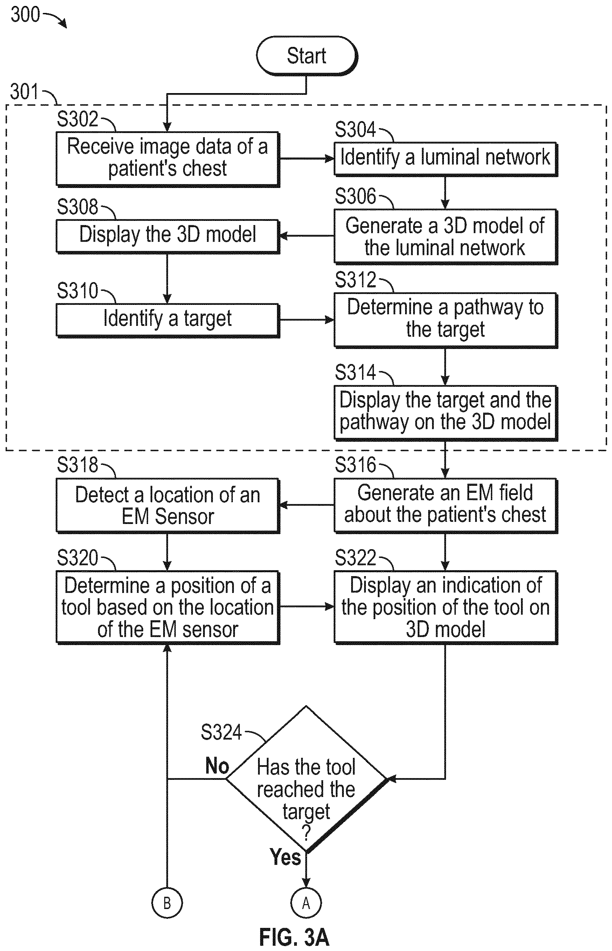

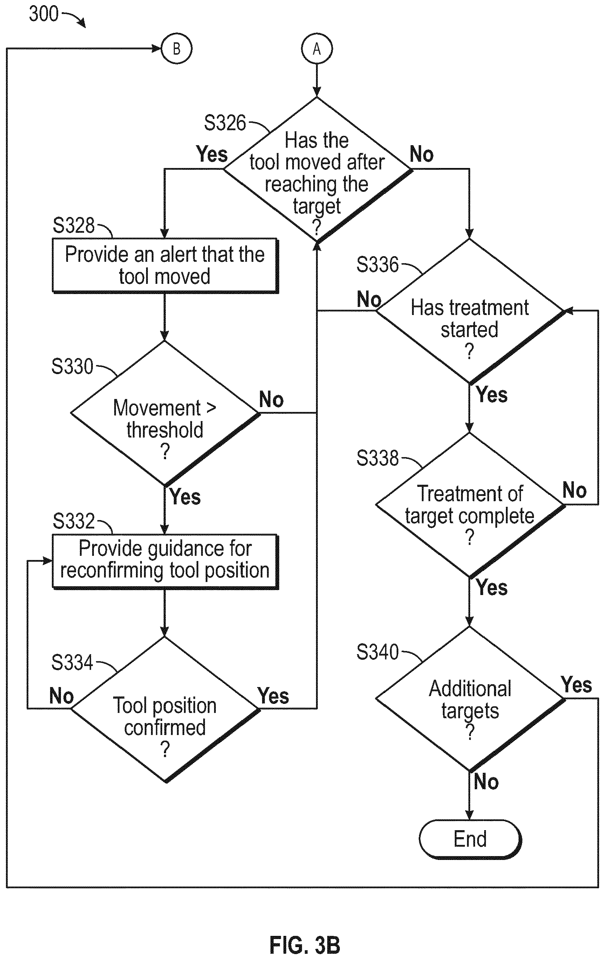

With reference to FIGs. 3A and 3B (collectively referred to as FIG. 3), there is shown a flowchart of an illustrative method 300 for identifying tool displacement during a surgical procedure, according to embodiments of the present disclosure. As described below, various subsections of method 300 may be directed at different embodiments. Thus, those skilled in the art will recognize that while presented and described as an ordered flow, various steps of method 300 may be repeated, omitted, and/or performed in a different order than described below. Additionally, while the description of method 300 below refers to computing device 80 performing various tasks, those skilled in the art will recognize that some or all of the tasks may be performed by computing device 80 in conjunction with execution of various applications, such as application 81, to assist and/or enable computing device 80 to perform the tasks.

Starting with FIG. 3A, an initial portion 301 of method 300 includes steps of the above-described planning phase of the treatment procedure. Thus, method 300 may start at step S302 where computing device 80 receives image data of the patient's chest. The image data may be acquired during a pre-procedure scan of the patient's body, or a relevant portion thereof, such as a CT scan, MRI scan, PET scan, CBCT scan, etc. Additionally or alternatively, the image data may be acquired during a scan, for example a CBCT scan, performed at the start of the treatment procedure.

After receiving the image data, computing device 80 processes the image data, at step S304, to identify a luminal network in the image data. The luminal network may include the patient's airways, blood vessels, and/or lymphatic vessels in the patient's lungs. Computing device 80 may further process the image data to identify other structures, such as the pleura and fissures of the patient's lungs, other organs and critical structures, and/or aberrant structures in and/or around the patient's lungs. Computing device 80 then, at step S306, generates a 3D model based on the processed image data, and displays the generated 3D model at step S308. For example, computing device 80 may cause display 206 to display the 3D model. In some embodiments, computing device 80 may further display the image data in conjunction with the 3D model.

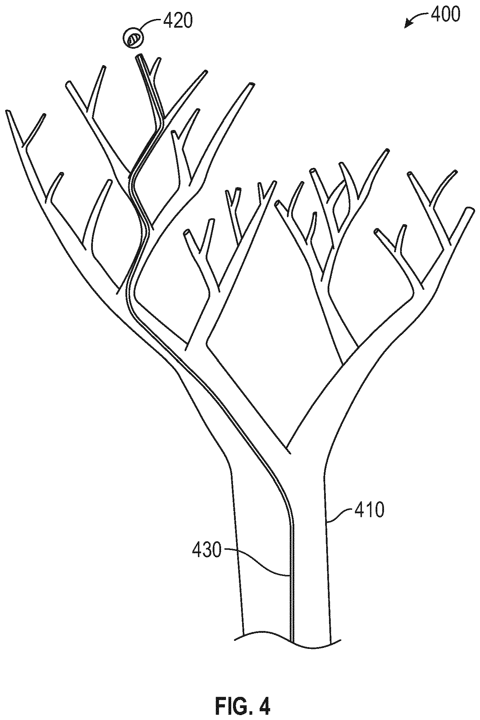

Next, at step S310, at least one treatment target is identified, either automatically by computing device 80, semi-automatically with input from the clinician, or manually by the clinician. For example, the at least one treatment target may be identified based on the image data and/or the 3D model. After identifying the treatment target, a target location representative of the identified location of the treatment target is marked on the 3D model, and computing device 80 determines a pathway between the patient's trachea and the target location via the patient's airways at step S312. As noted above, the pathway may include various portions, including at least one portion located inside the patient's airways running between the trachea and an exit point in an airway wall proximate the target location, and at least one portion located outside the patient's airways running from the exit point to the target location. The pathway represents a recommended route along which LG 92 or other tools including EM sensors 94, 95 should be navigated through the patient's airways and, as described further below, after reaching the exit point, through the tissue and space surrounding the patient's airways. Computing device 80 then displays the pathway and the target location on the 3D model at step S314. For example, as shown in FIG. 4 below, a view 400 of the 3D model may include an airway tree 410, an indicator of the target location 420, and a pathway 430 to the target location 420.

After steps S302-S314 are performed, the navigation phase of the treatment procedure commences. As an initial task, the 3D model must be registered to the actual airways of the patient's lungs. As described above, there are various methods of registration that may be used for this purpose, including the lung survey method described above. Alternatively, as also mentioned above, if the 3D model is generated based on image data, such as from a CBCT scan, acquired at the start of the treatment procedure, and the patient remains in substantially the same position on table 40 during the above-described planning phase, a lung survey may not be necessary, because computing device 80 may collect sufficient data points regarding the position of EM sensors 94, 95 in the patient's airways during the initial portion of the navigation phase to register the 3D model to the patient's airways while navigation is in progress.

To start this process, at step S316, EM field generator 76 generates an EM field about the patient's chest. EM sensors 94, 95, whether included in LG 92, tools 62, 64, and/or directly in EWC 96, is then inserted into the patient's airways and a location of EM sensors 94, 95 in the EM field is detected by tracking system 70 at step S318. The detected location of EM sensors 94, 95 is relayed to computing device 80 to determine, at step S320, a position of LG 92, tools 62, 64, and/or EWC 96 based on the detected location of EM sensors 94, 95. As mentioned herein, the detected position of EM sensors 94, 95 may be reflective of whichever tool EM sensors 94, 95 are included in, such as LG 92, tools 62, 64, and/or EWC 96, but for purpose of brevity and to ease this description, an example wherein LG 92 is used for navigation will be described hereinafter. However, those skilled in the art will realize that any of the other aforementioned tools and devices including EM sensors 94, 95 could be substituted for LG 92.

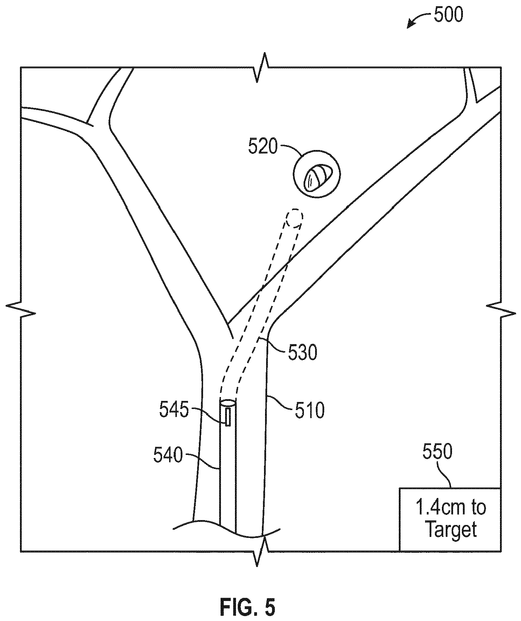

Next, at step S322, computing device 80 displays an indication of the determined position of LG 92 on the 3D model. For example, as shown in FIG. 5, a view 500 of the 3D model includes at least an airway tree 510, a tool indicator 540 (here representing LG 92), an indicator 545 of the determined position of EM sensors 94, 95, the target location 520, the pathway 530 to the target location, and guidance information 550, such as a distance indicator between EM sensor indicator 545 and target 520. Thereafter, at step S324, computing device 80 determines whether LG 92 has reached the target location. For example, computing device 80 may determine whether the detected position of EM sensors 94, 95 are within a predetermined distance from the location of the treatment target as marked in step S310. The predetermined distance may be selected by the clinician prior to the start of the treatment procedure, and may differ based on the clinician's preference and/or the type of treatment procedure being performed. If it is determined that LG 92 has reached the target location ("YES" at step S324), processing proceeds to step S326. Alternatively, if it is determined that LG 92 has not reached the target location ("NO" at step S324), processing returns to step S320. As such, steps S320 to S324 may be iteratively repeated while navigation is in progress to continually detect the location of EM sensors 94, 95 in the EM field, and determine and display a corresponding location of LG 92 on the 3D model until LG 92 reaches the treatment target.

Additionally, as noted above, the clinician may, at various times during the procedure, request that an intra-procedural scan be performed to verify the determined position of LG 92. For example, the clinician may request that a CBCT and/or fluoroscopic scan be performed when LG 92 reaches the exit point where the pathway moves from within the patient's airways to outside of the patient's airways. The clinician may also request that an additional scan be performed after LG 92 has been navigated through the airway wall and towards the target location outside of the patient's airways. Further, the clinician may request that an additional scan be performed to confirm the location of LG 92 when LG 92 is navigated proximate the target location, such as to confirm placement of LG 92 at the treatment target.

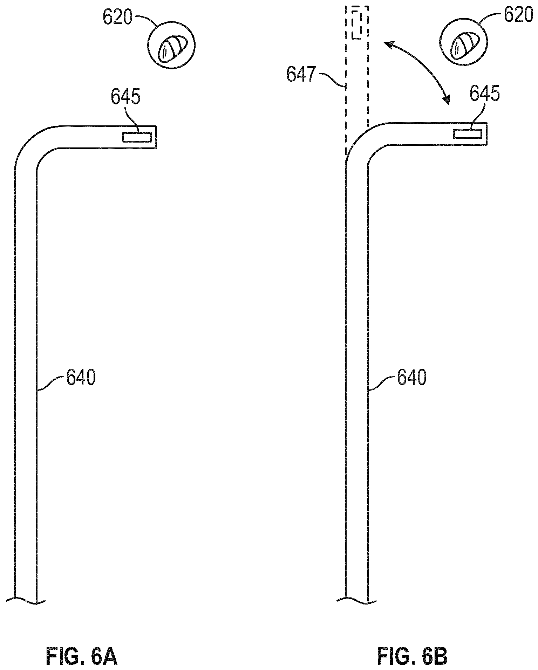

Turning now to FIG. 3B, at step S326, computing device 80 determines whether EWC 96 has moved after reaching the target location. For example, computing device 80 may determine whether EM sensor 95 has moved after reaching the target location. Additionally or alternatively, computing device 80 may determine, based on additional image data acquired during an intra-procedural scan, whether EWC 96 has moved after reaching the target location. As noted above, LG 92 may be used during the navigation phase of the treatment procedure to assist the clinician with placing EWC 96 at the target location. After LG 92 reaches the target location, LG 92 is removed from EWC 96 and another tool, such as tool 62, 64, is inserted into EWC 96 to perform treatment at the target location. However, some tools may be more rigid than LG 92, and thus may cause EWC 96 to move during insertion of tool 62, 64. For example, as shown in FIG. 6A, a distal portion of EWC 96, represented by tool indicator 640, may move relative to the target location 620 when tool 62, 64 is inserted. In the example shown in FIG. 6A, tool indicator 640 is positioned proximate target location 620, as determined based on a detected position of EM sensor 645. In the example shown in FIG. 6B, after tool 62, 64 is inserted into EWC 96, the distal portion of tool indicator 640 may move relative to the target location 620, as shown by moved portion 647.

In some embodiments, two or more intra-procedural scans may be performed during step S326 to determine whether EWC 96 has moved after reaching the target location. The two or more intra-procedural scans may be performed at different orientations or angles relative to the patient. For example, because radiographic images are typically displayed as two-dimensional (2D) images, movement of EWC 96 in the 3rd dimension relative to the 2D images may not be detectable by viewing 2D images. Thus, by performing the two or more intra-procedural scans at different orientations or angles relative to the patient, movement of EWC 96 in the 3rd dimension relative to the 2D images from one of the intra-procedural scans will be detectable in 2D images from another of the intra-procedural scans.

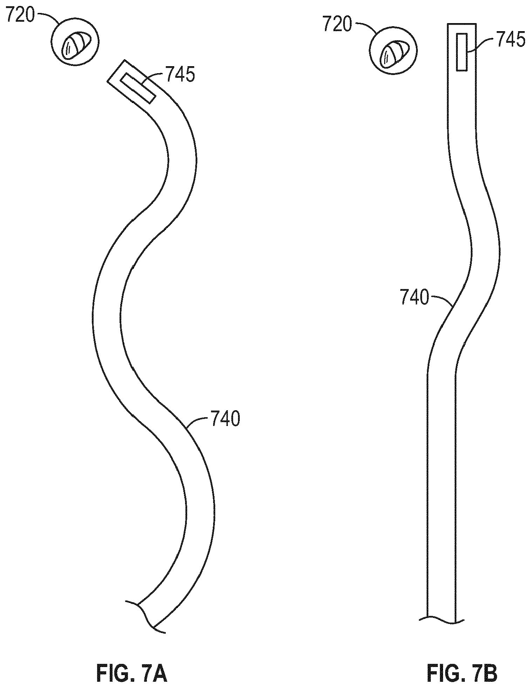

While the examples shown in FIGs. 6A and 6B are localized to the distal portion of EWC 96 to show a particular example of how the distal portion of EWC 96 may move relative to the target location, it should be noted that the movement is not limited to the distal portion of EWC 96. Particularly, as shown in the example of FIGs. 7A and 7B, movement may occur along the entire length of EWC 96. As shown in FIG. 7A, EWC 96, as represented by tool indicator 740, may not be disposed along a single axis (e.g. in a straight line) but rather, due to the tortuous nature of the patient's airways, will include multiple curves and/or deviations from a single axis as EWC 96 follows the patient's airways to the target location. As such, when a more rigid tool is inserted into EWC 96, EWC 96 may partially "straighten out" and some of the sharper curves may become less sharp, as shown in FIG. 7B. This is due to the more rigid tool causing the EWC 96 to at least partially displace the airways and/or parenchyma instead of conforming to the contours of the airways. Such displacement of the airways and/or parenchyma then causes EWC 96 to straighten out, as shown in FIG. 7B. Thus, straightening out of EWC 96 may cause EWC 96 to at least partially move relative to the target location. For example, the angle of approach of the distal portion of EWC 96 may change, and thus if EWC 96 was aligned with the target location before tool 62, 64 was inserted, the insertion of tool 62, 64 may cause EWC 96 to become misaligned with the target location. Additionally, the straightening out of EWC 96 may cause the distal portion of EWC 96 to move further (e.g. beyond) the position where the distal portion of EWC 96 was placed before LG 92 was removed. As such, computing device 80 may receive tracking data from tracking system 70 indicating that the EM sensor 95 coupled to EWC 96 has moved after LG 92 was removed from EWC 96, thus indicating that the distal portion of EWC 96 has moved. In some embodiments, one or more additional EM sensors 95 along the length of EWC 96, may indicate that one or more other portions of EWC 96 has moved when tool 62, 64 is inserted into EWC 96.

Further, in addition to the above-described movement of EWC 96 caused by insertion of tool 62, 64, EWC 96 may also be displaced by other events or due to other factors. For example, the respiration cycle of the patient, e.g. the expansion and contraction of the patient's chest, may cause EWC 96 to move relative to the position of the treatment target. Similarly, coughing, sneezing, or other respiratory events may also cause EWC 96 to move due to displacement of the patient's airways. Additionally, movement of the patient, both externally or internally (e.g. a heartbeat) may also cause EWC 96 to be displaced if the portion of the patient's airways in which EWC 96 is located is affected by the movement. Thus, reference sensors 74 positioned on the patient's chest, as tracked by EM tracking system 70, may indicate displacement of the patient's chest and/or other movement of the patient's body within the EM field. Computing device 80 may determine, based on data received from EM tracking system 70 indicating displacement of reference sensors 74, whether the displacement of reference sensors 74 correspond to movement of the patient's chest and/or body that may cause EWC 96 to move relative to the position of the treatment target. For example, if computing device 80 determines that the displacement of reference sensors 74 is above a threshold amount, and/or that the displacement of reference sensors 74 occur over a short period of time (e.g. in less than a predetermined amount of time), computing device 80 may determine that the displacement of reference sensors 74 indicates a cough, sneeze, or other respiratory events, or other movement of the patient's body that may cause EWC 96 to move relative to the position of the treatment target. Likewise, the clinician may unintentionally cause the EWC 96 to move by intentionally or accidentally moving bronchoscope 50, catheter guide assembly 90, and/or EWC 96. As such, if computing device 80 receives tracking data from tracking system 70 indicating that EM sensor 95 has moved after reaching the target location ("YES" at step S326), processing proceeds to step S328. Alternatively, if computing device 80 determines that EWC 96 and/or EM sensor 95 has not moved after reaching the target location ("NO" at step S326), processing proceeds to step S336.

At step S328, computing device 80 provides an alert that EWC 96 and/or EM sensor 95 has moved after reaching the target location. In embodiments, computing device 80 may cause display 206 to display an alert or cause another output device to provide an audible alert, such as a warning sound. Computing device 80 may provide the alert for any movement of EM sensor 95 above a minimum threshold (e.g. a threshold that is higher than the margin of error of EM tracking system 70).

Thereafter, at step S330, computing device 80 determines whether the movement of EWC 96 and/or EM sensor 95 is greater than a maximum threshold. The maximum threshold may be predetermined based on input provided by the clinician before the start of the treatment procedure, and/or may be automatically determined by computing device 80 based on the type of tool 62, 64 used and the type of procedure being performed. For example, during a biopsy procedure, precise placement of tool 62, 64 when obtaining a biopsy sample may be more important than placement of tool 62, 64 during an ablation procedure. Additionally, other structures in the proximity of the target location may require that placement of tool 62, 64 be more precise than may be required by the type of tool or type of procedure. For example, if critical structures are located adjacent or in close proximity to the target location, an ablation tool 64, which in other instances may not require such precise placement, may need to be placed with more precision to ensure that the zone of tissue to be ablated during the ablation procedure does not include the critical structure. Thus, the maximum threshold may be a larger distance, and thus allow for less-precise placement, during an ablation procedure than during a biopsy procedure. If computing device 80 determines that the movement of EWC 96 and/or EM sensor 95 is not greater than the maximum threshold ("NO" at step S330), processing returns to step S326. Alternatively, if computing device 80 determines that the movement of EWC 96 and/or EM sensor 95 is greater than the maximum threshold ("YES" at step S330), processing proceeds to step S332.

At step S332, computing device 80 provides guidance for reconfirming the position of EWC 96 and/or tool 62, 64. In embodiments, computing device 80 may cause display 206 to display instructions advising the clinician to remove tool 62, 64 from EWC 96 and reinsert LG 92 to reposition EWC 96 at the target location. In other embodiments, computing device 80 may provide instructions that the clinician should perform a CBCT scan or other type of imaging to reposition and/or confirm the placement of EWC 96 and/or tool 62, 64 at the target location.

Thereafter, at step S334, computing device 80 determines whether the placement of EWC 96 and/or EM sensor 95 at the target location has been confirmed. In embodiments, computing device 80 may determine that the placement of EWC 96 and/or EM sensor 95 at the target location has been confirmed based on tracking data received from tracking system 70 indicating that EM sensor 95 has been moved to a distance less than the maximum threshold from the target location. In other embodiments, computing device 80 may receive input from the clinician indicating that the placement of EWC 96 and/or EM sensor 95 has been confirmed. In yet further embodiments, computing device 80 may receive additional image data from a CBCT or other imaging modality, process the image data to identify EWC 96 and/or the target location, and determine based on the processing that EWC 96 is at the target location. If computing device 80 determines that the placement of EWC 96 and/or EM sensor 95 at the target location has not been confirmed ("NO" at step S334), processing returns to step S332. Alternatively, if computing device 80 determines that the placement of EWC 96 and/or EM sensor 95 at the target location has been confirmed ("YES" at step S334), processing returns to step S326.

At step S336, computing device 80 determines whether the treatment phase has started. The treatment phase starts when tool 62, 64 is used to perform treatment at the treatment target. In embodiments, computing device 80 may determine that the treatment phase has started based on a signal and/or data received from tool 62, 64 and/or generator 66 indicating that tool 62, 64 has been activated. Additionally or alternatively, computing device 80 may determine that the treatment phase has started based on input provided by the clinician. If computing device 80 determines that the treatment phase has not started ("NO" at step S326), processing returns to step S326. Alternatively, if computing device 80 determines that the treatment phase has started ("YES" at step S336), processing proceeds to step S338.

At step S338, computing device 80 determines whether the treatment of the current target has been completed. In embodiments, computing device 80 may determine that the treatment of the current target has been completed based on a signal and/or data received from tool 62, 64 and/or generator 66 indicating that tool 62, 64 is no longer being activated. Additionally or alternatively, in the case of an ablation procedure, computing device 80 may determine, based on a progress of the ablation, whether the ablation procedure has been completed. For example, computing device 80 may determine based on preconfigured time, power, and/or temperature settings for the ablation procedure that the ablation of the current target has been completed. If computing device 80 determines that the treatment of the current target has not been completed ("NO" at step S338), processing returns to step S336. Alternatively, if computing device 80 determines that the treatment of the current target has been completed ("YES" at step S338), processing proceeds to step S340.

At step S340, computing device 80 determines whether there are additional treatment targets remaining to be treated in the treatment procedure. For example, computing device 80 may determine whether additional targets were identified at step S310 and have not yet been treated. In some embodiments, computing device 80 may receive input from the clinician indicating that there are additional treatment targets remaining to be treated. If computing device 80 determines that there are additional treatment targets remaining to be treated ("YES" at step S340), processing returns to step S320, whereafter EWC 96 is navigated to another target location. Alternatively, if computing device 80 determines that there are no additional targets remaining to be treated, processing ends.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical system comprising:
   a surgical tool;
   an electromagnetic (EM) sensor;
   an EM navigation system including:
      an EM field generator configured to generate an EM field, and
      an EM tracking system configured to track the EM sensor within the EM field; and
   a computing device including a processor and a memory storing instructions which, when executed by the processor, cause the computing device to:
      receive a position of a target within the EM field,
      receive tracking data from the EM tracking system regarding a position of the EM sensor,
      determine that the surgical tool is placed proximate the target,
      determine that the surgical tool has moved after the surgical tool is placed proximate the target, and
      provide an alert.
2. The system according to paragraph 1, wherein the instructions, when executed by the processor, further cause the computing device to:
   determine that the movement of the surgical tool is greater than a predetermined threshold; and
   cause a display device to display guidance for confirming placement of the surgical tool.
3. The system according to paragraph 2, wherein the guidance for confirming placement of the surgical tool includes instructions to acquire image data of at least a portion of the patient's body.
4. The system according to paragraph 2, wherein the guidance for confirming placement of the surgical tool includes instructions to reposition the surgical tool.
5. The system according to paragraph 2, wherein the surgical tool is an extended working channel (EWC), and wherein the guidance for confirming placement of the surgical tool includes instructions to remove a treatment tool from the EWC and insert a tool including a second EM sensor into the EWC.
6. The system according to paragraph 2, wherein the predetermined threshold is based on a type of surgical procedure being performed.
7. The system according to paragraph 2, wherein the predetermined threshold is based on critical structures proximate the target.
8. The system according to paragraph 1, wherein the EM sensor is coupled to the surgical tool, and
   wherein determining that the surgical tool is placed proximate the target includes determining that the EM sensor is proximate the position of the target.
9. The system according to paragraph 1, wherein the EM sensor is coupled to the surgical tool, and
   wherein determining that the surgical tool has moved after the surgical tool is placed proximate the target includes determining that the EM sensor has moved after the surgical tool is placed proximate the target.
10. The system according to paragraph 1, wherein the EM sensor is positioned on the patient's chest.
11. The system according to paragraph 1, further comprising an extended working channel (EWC),
   wherein the EM sensor is coupled to the EWC,
   wherein the surgical tool is inserted into the EWC, and
   wherein determining that the surgical tool is placed proximate the target includes determining that the EM sensor is proximate the position of the target.
12. The system according to paragraph 11, wherein determining that the surgical tool has moved after the surgical tool is placed proximate the target includes determining that the EM sensor has moved after the surgical tool is placed proximate the target.
13. The system according to paragraph 1, wherein the instructions, when executed by the processor, further cause the computing device to:
   receive image data of at least a portion of a patient's body;
   cause a display device to display the image data;
   cause the display device to display an indication of the position of the EM sensor on the image data.
14. The system according to paragraph 1, wherein the instructions, when executed by the processor, further cause the computing device to receive image data of at least a portion of a patient's body,
   wherein the determination that the surgical tool has moved after the surgical tool is placed proximate the target is based on the image data.
15. The system according to paragraph 1, wherein the instructions, when executed by the processor, further cause the computing device to cause a display device to display an indication of movement of the surgical tool when it is determined that the surgical tool has moved after the surgical tool is placed proximate the target.
16. The system according to paragraph 1, wherein the instructions, when executed by the processor, further cause the computing device to:
   receive image data of at least a portion of a patient's body;
   generate a three-dimensional (3D) model of the portion of the patient's body based on the image data;
   cause a display device to display the 3D model; and
   cause the display device to display an indication of the position of the EM sensor on the 3D model.
17. The system according to paragraph 16, wherein the instructions, when executed by the processor, further cause the computing device to:
   cause the display device to display an indication of the position of the target on the 3D model; and
   cause the display device to display an indication of a distance between a position of the surgical tool and the position of the target.
18. The system according to paragraph 17, wherein the position of the surgical tool is determined based on the position of the EM sensor.
19. The system according to paragraph 17, wherein the position of the surgical tool is determined based on image data.
20. The system according to paragraph 1, wherein providing an alert includes causing a display device to display a visual alert.
21. The system according to paragraph 1, wherein providing an alert includes emitting an audible alert.
22. The system according to paragraph 1, wherein determining that the surgical tool has moved after the surgical tool is placed proximate the target includes determining that the movement of the surgical tool is greater than a predetermined threshold.

## Claims

1. A surgical system comprising:
a surgical tool;
an electromagnetic (EM) sensor;
an EM navigation system including:
an EM field generator configured to generate an EM field, and
an EM tracking system configured to track the EM sensor within the EM field; and
a computing device including a processor and a memory storing instructions which, when executed by the processor, cause the computing device to:
receive a position of a target within the EM field,
receive tracking data from the EM tracking system regarding a position of the EM sensor,
determine that the surgical tool is placed proximate the target,
determine that the surgical tool has moved after the surgical tool is placed proximate the target, and
provide an alert.

2. The system according to claim 1, wherein the instructions, when executed by the processor, further cause the computing device to:
determine that the movement of the surgical tool is greater than a predetermined threshold; and
cause a display device to display guidance for confirming placement of the surgical tool.

3. The system according to claim 2, wherein the guidance for confirming placement of the surgical tool includes instructions to acquire image data of at least a portion of the patient's body; and/or wherein the guidance for confirming placement of the surgical tool includes instructions to reposition the surgical tool.

4. The system according to claim 2, wherein the surgical tool is an extended working channel (EWC), and wherein the guidance for confirming placement of the surgical tool includes instructions to remove a treatment tool from the EWC and insert a tool including a second EM sensor into the EWC.

5. The system according to claim 2, wherein the predetermined threshold is based on a type of surgical procedure being performed; and/or wherein the predetermined threshold is based on critical structures proximate the target.

6. The system according to any preceding claim, wherein the EM sensor is coupled to the surgical tool, and
wherein determining that the surgical tool is placed proximate the target includes determining that the EM sensor is proximate the position of the target.

7. The system according to any preceding claim, wherein the EM sensor is coupled to the surgical tool, and
wherein determining that the surgical tool has moved after the surgical tool is placed proximate the target includes determining that the EM sensor has moved after the surgical tool is placed proximate the target; and/or wherein the EM sensor is positioned on the patient's chest.

8. The system according to any preceding claim, further comprising an extended working channel (EWC),
wherein the EM sensor is coupled to the EWC,
wherein the surgical tool is inserted into the EWC, and
wherein determining that the surgical tool is placed proximate the target includes determining that the EM sensor is proximate the position of the target; preferably, wherein determining that the surgical tool has moved after the surgical tool is placed proximate the target includes determining that the EM sensor has moved after the surgical tool is placed proximate the target.

9. The system according to any preceding claim, wherein the instructions, when executed by the processor, further cause the computing device to:
receive image data of at least a portion of a patient's body;
cause a display device to display the image data;
cause the display device to display an indication of the position of the EM sensor on the image data.

10. The system according to claim 9, wherein the instructions, when executed by the processor, further cause the computing device to receive image data of at least a portion of a patient's body,
wherein the determination that the surgical tool has moved after the surgical tool is placed proximate the target is based on the image data; and/or wherein the instructions, when executed by the processor, further cause the computing device to cause a display device to display an indication of movement of the surgical tool when it is determined that the surgical tool has moved after the surgical tool is placed proximate the target.

11. The system according to claim 9 or claim 10, wherein the instructions, when executed by the processor, further cause the computing device to:
receive image data of at least a portion of a patient's body;
generate a three-dimensional (3D) model of the portion of the patient's body based on the image data;
cause a display device to display the 3D model; and
cause the display device to display an indication of the position of the EM sensor on the 3D model; preferably wherein the instructions, when executed by the processor, further cause the computing device to:
cause the display device to display an indication of the position of the target on the 3D model; and
cause the display device to display an indication of a distance between a position of the surgical tool and the position of the target.

12. The system according to claim 11, wherein the position of the surgical tool is determined based on the position of the EM sensor; and/or wherein the position of the surgical tool is determined based on image data.

13. The system according to any preceding claim, wherein providing an alert includes causing a display device to display a visual alert; and/or wherein providing an alert includes emitting an audible alert.

14. The system according to any preceding claim, wherein determining that the surgical tool has moved after the surgical tool is placed proximate the target includes determining that the movement of the surgical tool is greater than a predetermined threshold.
